# EUROPEAN PATENT SPECIFICATION

(11) **EP 1 231 961 B1**
(45) Date of publication and mention of the grant of the patent: **12.07.2006**
(21) Application number: 00982218.0
(22) Date of filing: 22.11.2000
(51) Int. Cl.: A61M 5/30, A61M 5/48

(54) **INJECTOR ASSEMBLY WITH DRIVING MEANS AND LOCKING MEANS**
INJEKTORANORDNUNG MIT ANTRIEBS- UND VERRIEGELUNGSMITTELN
ENSEMBLE INJECTEUR MUNI D'ELEMENTS D'ACTIONNEMENT ET DE VERROUILLAGE

(30) Priority: 23.11.1999 RU 99124267; 10.10.2000 US 685499; 10.10.2000 US 685633; 21.11.2000 US 717548; 21.11.2000 US 717559
(43) Date of publication of application: 21.08.2002
(73) Proprietor: Felton International, Inc., Lenexa, KS 66214 (US)
(72) Inventor: SMOLYAROV, Boris V., Voronezh, 394051 (RU); ROGATCHEV, Victor T., Voronezh, 394062 (RU)
(74) Representative: Schnappauf, Georg
(86) International application number: PCT/US2000/032186
(87) International publication number: WO 2001/037907

(56) References cited:
- EP-A- 0 347 190
- EP-A- 0 956 873
- US-A- 4 007 739
- US-A- 4 266 541
- US-A- 4 426 024
- US-A- 4 913 699
- US-A- 5 176 645
- US-A- 5 520 639
- US-A- 5 851 198
- US-A- 5 865 795
- US-A- 6 102 896

## Description

### Technical Field of the Invention:

The invention relates to a jet injector device having a locking means that prevents a driving means from expelling a substance before a predetermined amount of pressure has been achieved within the device.

A jet injector assembly as defined in the preamble of claim 1 is disclosed in US-A-5 520 639.

### Background of the Invention:

The traditional needleless injectors include the basic design: a housing with an inner power unit, a medication unit, and a nozzle. The function of the power unit is to pump the medication into an under-plunger cavity of the medication unit chamber and to expel the medication through the nozzle.

Some needleless injectors such as those disclosed in U.S. Patent number 5,851,198 to Castellano et al, U.S. Patent number 5,520,639 to Peterson et al and U.S. Patent number 4,913,699 to Parsons are powered by a pneumatic drive. These injectors may include an injection chamber having a piston and nozzle, a pneumatic cylinder having a power pneumatic piston, a gas distribution unit, a pipeline for the supply of compressed air into the pneumatic cylinder, and an exhaust for the release of compressed air into the atmosphere. Significant disadvantages prevail in this type of injector. First, the time for increasing a pressure to drive the pneumatic piston is relatively long. A dead volume in the injector cavity requires time to fill and achieve the required pressure. This initially allows jet formation with insufficient kinetic energy to properly pierce the subject's skin and results in poor overall injection quality both in terms of the depth (transdermal, subcutaneous, or intramuscular) and the amount of medication delivered. To this end, a device that prevents the driving means from expelling the medicine before the required amount of pressure has built (as is the case in the device of U.S. Patent number 5,865,795 to Schift et al) is needed.

Another disadvantage of the described needleless injector is the need to return the power piston and other moveable parts to their initial position. Typically, this is accomplished using a mechanical spring. Mechanical springs increase the dimensions and mass of the jet injector and are a common mode of failure.

US Patent No. 6,280,421 (equivalent to EP 0 956 873) describes a needle requiring auto-injection device. The device including a driving means in the for of a piston for injecting a substance into a subject, a container from which a product is dispensed by advancing the piston, and a drive unit including a driven member which is advanced by the mechanism of a compressed spring when the force of the spring is released from the locking means utilising a means for releasing in the form of a release tab.

US Patent No. 5,865,795 also describes an injector assembly. The assembly requires a needle. The assembly has a driving means in the form of a piston for injecting a substance into a subject. Locking means are provided for retaining the piston. Means are also provided for releasing the retained driving means from the locking means to effect injection.

EP 0 347 190 A1 discloses a needleless injector which includes driving means in the form of a piston for injecting a substance into a subject. The driving means is retained in position until sensing means detect sufficient vacuum between the device and a subject to be injected, whereupon when sufficient vacuum is detected the driving means is actuated to effect injection.

US Patent No. 6,102,896 published after the priority date of the present invention discloses a single use injector which utilises hand pressure to effect injection by employing a snap mechanism which relies on sudden failure under manual pressure to deliver the required pressure for injection.

Accordingly, there is a need in the art of needleless injector devices to solve the problem of early injection. More particularly, there is a need for an injector that increases the quality of an injection, decreases the loss of medicine on the skin's surface, and decreases the dimensions and mass of a jet injector.

### Summary of the Invention:

The foregoing problems are solved and a technical advance is achieved by the present invention as defined in claim 1. Disclosed is an injector assembly having driving means for injecting a substance into a subject and locking means for retaining the driving means. More particularly, the present invention relates to an injector assembly comprising a piston assembly having a piston and a spring, a lock assembly having at least one roller, at least one roller retainer, and an annular groove, the lock assembly configured to engage the piston assembly, an injection chamber, wherein the piston assembly is configured to drive a substance out of the injection chamber, and a pneumatic pressure unit detachably attached to the piston assembly by at least one path.

### Brief Description of the Drawings:

FIG. 1 is a cross-sectional view of one embodiment of the invention.
FIG. 2 is a cross-sectional view demonstrating the driving assembly and lock assembly of the present invention.
FIG. 3a is a cross-sectional view demonstrating a pneumatic power unit of the present invention in a position prior to injection.
FIG. 3b is a cross-sectional view demonstrating a pneumatic power unit of the present invention in a position after injection.
FIG. 4 is a cross-sectional view of another embodiment of the present invention, particularly the dosing unit.
FIG. 5 is a cross-sectional view demonstrating another detail of the present invention, particularly the protective cap.

### Detailed Description of the Invention:

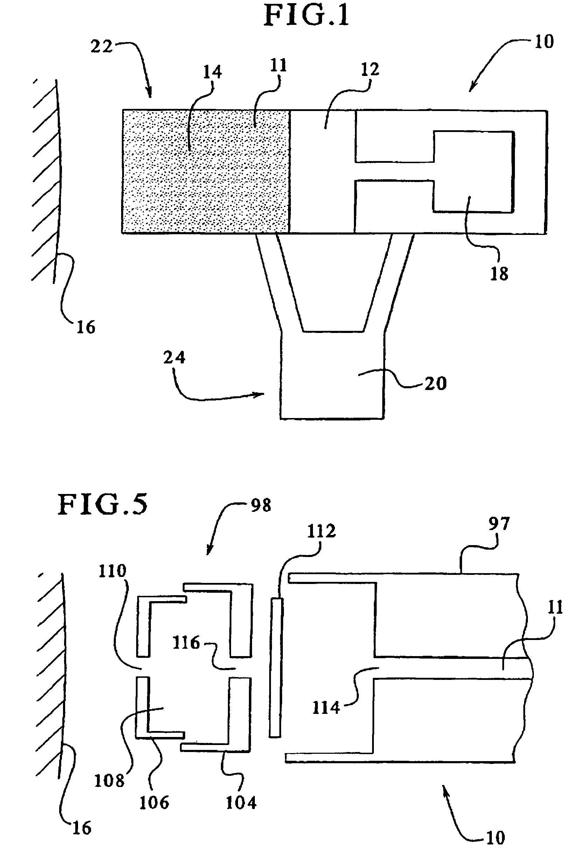

FIG. 1 depicts an injector assembly 10 having an injection chamber 11, a driving means 12 for injecting a substance 14 into a subject 16, and a locking means 18 for retaining the driving means 12. Injector assembly 10 may further include a means for releasing 20 the driving means 12 from the locking means 18. Injector assembly 10 may comprise one or more components housing the driving means 12, the locking means 18, and the means for relcasing 20. In one embodiment the driving means 12 and the locking means 18 are located in a first component 22 and the means for releasing 20 is located in a second component 24. The term "located in" encompasses all variations of the term, including but not limited to, being partially or completely located in a specified area.

Many types of driving means 12 may be employed in the present invention. The driving means 12 can include those means known in the art and can further include, but is not limited to, pistons, gears, rods, springs, work gears, screws, electromagnets, optical components, and jacks. The driving means 12 may also include various driving mechanisms, such as pneumatics, hydraulics, or manual drives. In addition, the driving means 12 may include phase change materials or other shape memory materials, such as those materials that change size or shape due to temperature application. One such material is Nitinol, which allows for size or shape transformation in its austenite and martensite states. Accordingly, the driving means 12 is meant to include not only the structures described herein, but also, any acts or materials described herein, and also include any equivalent structures, equivalent acts, or equivalent materials; or structural equivalents, act equivalents, or material equivalents, to those described herein.

Similarly, many types of locking means 18 may be employed in the present invention. The locking means 18 can include those means known in the art and can further include, but is not limited to, tongue and groove, rollers and retainers, notches, screw types with threading, electromagnetic devices, and restricto-magnetic devices. Accordingly, the locking means 18 is meant to include not only the structures described herein, but also, any acts or materials described herein, and also include any equivalent structures, equivalent acts, or equivalent materials; or structural equivalents, act equivalents, or material equivalents, to those described herein.

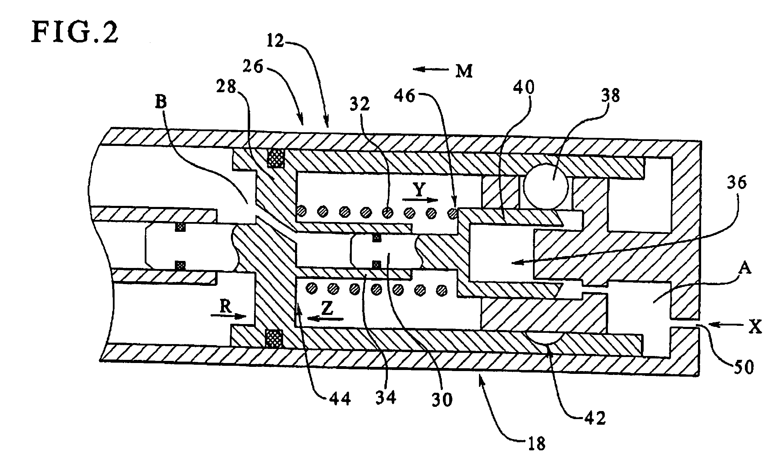

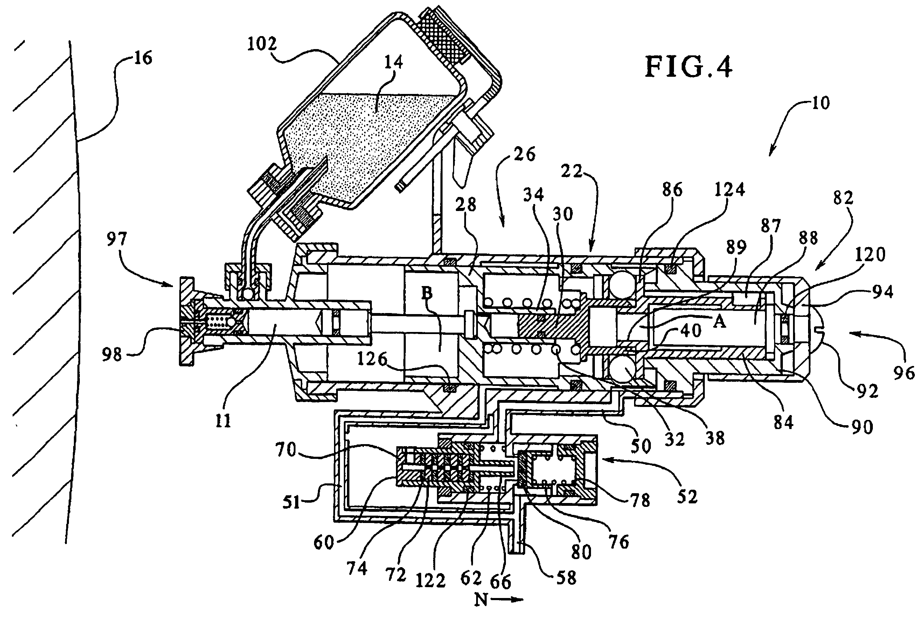

FIG. 2 depicts another embodiment of the invention. The driving means 12 comprises a driving assembly 26 having a piston 28, a piston rod 30, and a spring 32. The piston rod 30 glides through a cylinder 34 formed within the piston 28. The locking means 18 comprises a lock assembly 36 having at least one roller 38, at least one roller retainer 40, and annular groove 42. Annular groove 42 forms an internal toroid surface inside piston 28 and retains piston 28 in a locked position until the injection takes place. The embodiment in FIG. 2 depicts a lock assembly 36 having one roller 38, one roller retainer 40, and annular groove 42. It is to be understood by one skilled in the art that only one roller 38 and one roller retainer 40 is necessary to carry out the purpose of the invention; however, the lock assembly 36 may comprise one or more rollers 38 and roller retainers 40, as depicted in FIG. 4. The spring 32 of driving assembly 26 is situated between the piston 28 and the roller retainer 40. A proximal end 44 of the spring 32 abuts piston the 28 and a distal end 46 of the spring 32 abuts the roller retainer 40 when the driving assembly 26 is in a locked position. The piston rod 30 is manufactured jointly with the roller retainer 40. The roller retainer 40 holds and releases the roller 38 in the annular groove 42 of the piston 28.

Any type of means for releasing 20 may be employed in the invention including those means known in the art and further including, but not limited to, pneumatic power units, hydraulic power units, manual drives, cable connections, electro-mechanical devices, computer sources, or any combination thereof. Accordingly, the means for releasing 20 is meant to include not only the structures described herein, but also, any acts or materials described herein, and also include any equivalent structures, equivalent acts, or equivalent materials; or structural equivalents, act equivalents, or material equivalents, to those described herein.

In one embodiment, as shown in FIG. 4, the means for releasing 20 the driving means 12 comprises a power unit 52 and at least one path 50. At least one path 50 detachably attaches power unit 52 to first component 22 to provide fluid communication between the power unit 52 and the first component 22. More than one path may provide communication between the power unit 52 and the first component 22. The power unit 52 communicates with the first component 22 by applying a pressure to driving assembly 26. The power unit 52 may be powered by pneumatic pressure or hydraulic pressure, or any combination thereof.

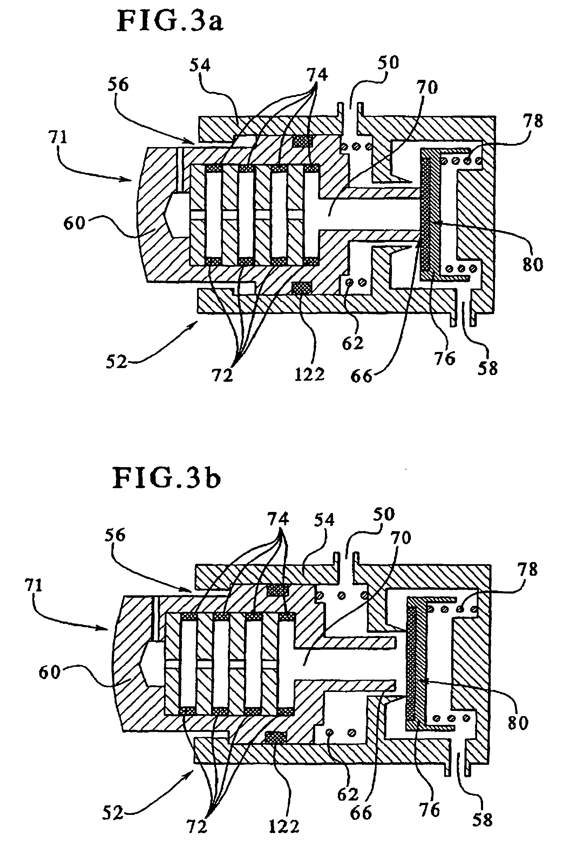

FIGs. 3a and 3b depict a pneumatic pressure power unit 52. Pneumatic power unit 52 modulates pneumatic energy supplied by power unit 52 to path 50. The pneumatic pressure unit 52 comprises a body 54 having a proximal end bore 56 and a distal end bore 58. A button 60 seats within the proximal end bore 56. The button 60 activates the pneumatic pressure unit 52 to release pressure in the form of compressed air. The button 60 is biased by a button spring 62 and is sealed by a ring 122. In one embodiment, the button 60 is hollow and comprises an inner end 66 and a channel 70. The channel 70 connects to the atmosphere outside of the injector assembly 10. The channel 70 serves as an exhaust assembly 71 further having several gaskets 74 separated by orifice plates 72 to suppress noise produced by the pneumatic pressure unit 52 upon injection of the substance 14 (not shown in FIGs. 4a and 4b). Compressed air is supplied through the distal end bore 58. An inlet valve 76 biased by a valve spring 78 controls the destination of the compressed air from distal end bore 58. A sealing element 80 seats between the inner end 66 of the button 60 and the inlet valve 76 and moves with the inlet valve 76 upon compression of the button spring 62 or release of the valve spring 78.

In one embodiment, depicted in FIG. 4, the pneumatic power unit 52 communicates with the first component 22 through the path 50 and the path 51. The path 50 supplies pressure to a cavity A near the piston rod 30. The inlet valve 76 controls the supply of compressed air to the path 50. The path 51 supplies pressure to a cavity B near the piston 28. The distinctions between these two paths and the function of the path 50 and the path 51 are described in more detail below.

FIG. 4 demonstrates another embodiment of the present invention. Shown is the first component 22 having a dosing unit 82 for adjusting the delivery of a predetermined amount of the substance 14 (FIG. 1). A guiding cylinder 84 seated within the dosing unit 82 includes a separator 86 abutting the rollers 38, a key 87, and a bore 88. In one embodiment, the bore 88 is a threaded bore. The separator 86 includes at least one radial hole, or pocket, (not shown) having a radius of a sufficient size to allow rollers 38 to seat within the separator 86 for maximum holding capacity. Limiter 89 protrudes from separator 86 to prevent the rollers 38 from falling out of the radial holes. The guiding cylinder 84 is movable within a cylindrical wall 90 of injector assembly 10. An adjustment screw 92 fits in the bore 88 to rotate the guiding cylinder 84 within the cylindrical wall 90. In one embodiment, the key 87 insures that the guiding cylinder 84 only moves axially relative to the cylindrical wall 90 as the adjustment screw 92 is rotated. A bushing 94 at a rearward end 96 of injector assembly 10 prevents axial movement of the adjustment screw 92. The bushing 94 guides the adjustment screw 92 to adjust the position of the separator 86 and thereby the amount of the substance 14 to be delivered to the subject 16.

Rings seal the injector assembly 10 at predetermined locations to prevent leakage of the substance 14 or the fluid or gas employed to create pressure, as depicted in FIG. 4 by rings 120, 122, 124, and 126, for example. In one embodiment, rings 120, 122, 124, and 126 are comprised of a polymeric material. In another embodiment, rings 120, 122, 124, and 126 provide a hermetic seal.

FIGs. 4 and 5 depict another detail of the invention. A protective cap 98 is shown disposed near a front end 97 of injector assembly 10. The protective cap 98 can be detachably attached to the front end 97 using conventional techniques, such as friction fits, bayonet fixing, male-female receptacles, or the like. The protective cap 98 is shown having a baffle 104 and an insert 106. The insert 106 can be adapted to form an insert reservoir 108. Insert 106 also has an insert distal orifice 110. Insert 106 can be adapted to fit with baffle 104 such that the insert 106 provides an additional benefit of back splash protection. As shown in this particular non-limiting embodiment, a protective layer 112 is generally located between, either partially or completely, the baffle 104 and an injector orifice 114. In this configuration, the substance will exit the injector orifice 114, penetrate through the layer 112, and exit through the baffle orifice 116 and insert distal orifice 110 to impact the subject 16. Other embodiments of the protective cap 98 are described in U.S. Patent Application Serial No. 09/685,499, filed Oct. 10, 2000.

Referring to FIG. 2, the driving assembly 26 exists within injector assembly 10 in either a resting state or an activated state. The driving assembly 26 is in the resting state when no external pressure in the direction depicted by arrow X is applied to the driving assembly 26. In the resting state, however, a force in the direction Y may be created by the driving assembly 26 and the lock assembly 36. The driving assembly 26 is in the activated state when the external pressure in the direction X is applied to driving assembly 26. When the driving assembly 26 is in the resting state, the driving assembly 26 may be in either a locked position or an unlocked position.

Two resting states and two activated states combine to inject the substance 14 into the subject 16. In the first resting state, the driving assembly 26 is in the locked position and is subject to the spring force in the direction Y. The force in the direction Y is created by the compression of the spring 32 between the piston 28 and the roller retainers 40 when the driving assembly 26 is in the locked position. A first activation state begins when the pressure indicated by the arrow X from the power unit 52 begins to fill cavity A near the piston rod 30. The force in the direction Y is released during the second resting state, which occurs when the pressure in the direction of arrow X reaches a predetermined amount. In the second resting state, no pressures act upon driving assembly 26. A second activated state begins when pressure in the direction R from the pneumatic pressure unit 52 begins to fill cavity B near the piston 28. The force in the direction Z is recreated when the driving assembly 26 enters into the second activated state. Once the pressure indicated by the arrow R reaches a predetermined amount to overcome the force in the direction Z during the second activated state, the driving assembly 26 returns to the first resting state in a locked position. The "predetermined amount of pressure" is defined as the amount of pressure sufficient to overcome the forces indicated by arrows Y and Z created by spring 32. In one embodiment, the predetermined amount of pressure is equal to about 70% of the operation pressure of the compressed gas.

In FIG. 2, the driving assembly 26 rests in the locked position in the first resting state. When the driving assembly 26 is in the locked position, the roller 38 seats in the annular groove 42 of the piston 28. The seating of the roller 38 into groove 42 prevents piston 28 from moving in the direction indicated by arrow M despite the torsion force of spring 32. The driving assembly 26 remains in this locked position of the first resting state until pressure indicated by the arrow X generated from the power unit 52 and supplied through the path 50 builds to the predetermined amount against the piston rod 30 in the cavity A during the first activated state. This occurs when a user presses the button 60 of the pneumatic pressure unit 52, as depicted in FIGs. 3a and 4. When the button 60 is depressed, the inner end 66 pushes against the sealing element 80 and the inlet valve 76, thereby closing channel 70 and opening the path 50 from the distal end bore 58 and releasing compressed air to the cavity A. Referring back to FIG. 2, when the predetermined amount of the pressure indicated by the arrow X is built against the piston rod 30, the roller retainer 40 pushes against the spring 32 in the direction M. As a result, the roller 38 rolls towards the center axis of the injector assembly 10 behind the roller retainer 40 causing the piston 28 to release from the roller 38 and to also move in the direction M under the pressure indicated by the arrow X from the power unit 52 and the force indicated by the arrow Z of the spring 32. The movement of the piston 28 in the M direction causes the substance 14 to propel through and exit the injection chamber 11 into the subject 16.

Referring to FIG. 3b and 4, when the injection is completed, the button 60 returns to its original released position under the force of the button spring 62. Under the action of the valve spring 78, the inlet valve 76 closes and interrupts the supply of compressed air into the cavity A. Then the inner end 66 opens to the path 50 thereby connecting the cavity A and the channel 70 to release the compressed air to the outside atmosphere. Air from the cavity A and the channel 70 exhausts to the outside atmosphere by passing through several gaskets 74 and orifice plates 72. Thus, the noise produced by the injection and exhaust of compressed air is suppressed.

Referring back to FIG. 2, once the piston 28 reaches the farthest distance in the M direction, the driving assembly 26 is in the unlocked position and enters into the second resting state. At this point, no pressure or force acts upon the driving assembly 26. The driving assembly 26 remains in the second resting state until the power unit 52 supplies the predetermined amount of the pressure indicated by the arrow R. As the compressed air exhausts from the channel 70, the inlet valve 76 and sealing element 80 prevent new compressed air supplied from the distal end bore 58 from travelling through the path 50 to the cavity A. Therefore, the compressed air bypasses through the path 51 to the cavity B. While the pressure indicated by the arrow R builds against the piston 28 in the cavity B during the second activated state, the piston 28 approaches the spring 32. When a predetermined amount of the pressure indicated by the arrow R builds against the piston 28, the spring 32 pushes the roller retainer 40 in the direction N toward the roller 38. Hence the roller retainer 40 pushes the roller 38 away from the center axis to secure the roller 38 into the annular groove 42 of the piston 28. At this point, the driving assembly 26 returns to the locked position of the first resting state.

In one embodiment, depicted in FIG. 4, as the driving assembly 26 travels in the direction N, a vacuum is created in the injection chamber 11. The vacuum draws the substance 14 from a vial 102 into the injection chamber 11 to prepare the injector assembly 10 for another injection. This mechanism and apparatus are described in further detail in U.S. Patent Application Serial No. 09/685,633, filed Oct. 10, 2000.

In yet another embodiment of the present invention as illustrated in FIG. 3, the dosage amount of the substance 14 can be adjusted by rotating the bushing 94 and the adjustment screw 92. Rotation of the adjustment screw 92 displaces the separator 86 along a horizontal axis. Displacement of the separator 86 can either increase or decrease the size of the injection chamber 11 by changing the motion length of piston 28. For example, clockwise rotation of the bushing 94 may cause the separator 86 to move towards the front end 97 of the injector assembly 10 thereby decreasing the size of the injection chamber 11; whereas counterclockwise rotation of the bushing 94 may cause the separator 86 to move towards the rearward end 96 of the injection assembly 10, thereby increasing the size of the injection chamber 11.

It should be understood that the foregoing relates only to a limited number of embodiments that have been provided for illustration purposes only. It is intended that the scope of invention is defined by the appended claims and that modifications to the embodiments above may be made that do not depart from the scope of the claims.

## Claims

1. A jet injector assembly (10), comprising:
(a) driving means (12) for injecting a substance (14) into a subject (16);
(b) locking means (18) for retaining the driving means (12); and
(c) means for releasing (20) the retained driving means (12) from the locking means (18);
the means for releasing (20) the retained driving means (12) from the locking means comprises a power unit (52) which comprises (a) at least one hydraulic or pneumatic pressure unit (b) a first path (50), which (50) provides fluid communication of hydraulic or pneumatic pressure from the power unit (52) to the locking means (18) wherein the locking means (18) is configured to release the driving means (12) when the power unit (52) builds a predetermined amount of hydraulic or pneumatic pressure in the first path (50), **characterised by**
(c) a second path (51) which provides fluid communication of hydraulic or pneumatic pressure from the power unit (52) to the driving means (12) wherein the driving means (12) is returned to a locked position to be released.

2. The injector assembly of claim 1, wherein the driving means (12) comprises a piston (28).

3. The injector assembly of claim 1 or claim 2, wherein the driving means comprises a spring (32).

4. The injector assembly of claim any preceding claim, wherein the driving means comprises a piston (28) and a spring (32).

5. The injector assembly of any preceding claim, wherein the locking means (18) comprises at least one roller (38).

6. The injector assembly of any preceding claim, wherein the locking means (18) comprises at least one roller retainer (46).

7. The injector assembly of any preceding claim, wherein the locking means (18) comprises an annular groove (42).

8. The injector assembly of any preceding claim, wherein the locking means (18) comprises at least one roller (38), at least one roller retainer (46), and an annular groove (42).

9. The injector assembly of any preceding claim, further comprising a first component (22).

10. The injector assembly of claim 9, wherein the driving means (12) and the locking means (18) are located in the first component (22).

11. The injector assembly of any preceding claim, wherein the locking means (18) is further configured to engage the driving means (12) in at least one of a locked position and an unlocked position.

12. The injector assembly of any one of claims 8 to 11, wherein the at least one roller retainer (46) is adapted to hold the at least one roller (38) in the annular groove (42) in a locked position.

13. The injector assembly of any preceding claim, wherein the power unit (52) is adapted to apply a pressure to the driving means (12).

14. An injector assembly according to claim 9 or claim 10 wherein the first component (22) comprises:
(i) a piston assembly comprising the piston (28) and the spring (32);
(ii) a lock assembly (18) comprising at least one roller (38), at least one roller retainer (46), and an annular groove (42), the lock assembly configured to engage the piston assembly; and
(iii) an injection chamber (11),
wherein the piston assembly is configured to drive a substance out of the injection chamber (11).

15. An injection assembly according to claim 14 wherein a pneumatic pressure unit (52) is detachably attached to the first component by at least one path (50).

16. The injector assembly of any one of claims 7 to 15, wherein the piston (28) further comprises the annular groove (42).

17. The injector assembly of any one of claims 7 to 16, wherein the at least one roller retainer (46) is adapted to hold the at least one roller (38) in the annular groove (42).

18. The injector assembly of any one of claims 15 to 17, wherein the pneumatic pressure unit (52) is adapted to apply a pressure to the piston assembly.

19. The injector assembly of any one of claims 15 to 18, wherein the pneumatic pressure unit (52) comprises an exhaust assembly (71) adapted to release compressed air from the pneumatic pressure unit.

20. The injector assembly of claim 19, wherein the exhaust assembly (71) comprises a channel (70), at least one gasket (74), and at least one orifice plate (72).

21. The injector assembly of any one of claims 14 to 20, wherein the first component (22) further comprises a protective cap (98).

22. The injector assembly of any one of claims 14 to 21, wherein the first component (22) further comprises a dosing unit (82) configured to adjust the delivery of a predetermined amount of a substance.

23. The injector assembly of any one of claims 13 to 22, wherein the predetermined amount of pressure is at least approximately 70% of the operation pressure of a compressed gas.

24. A method of preventing premature injection of a substance from an injector assembly, wherein the injector assembly is the injector assembly of claim 1, comprising:
(a) locking a driving assembly (12) into a lock assembly (18);
(b) increasing pressure from a pneumatic power unit (52) against the driving assembly (12);
(c) retaining the driving assembly (12) in the lock assembly (18) until a predetermined amount of pressure from the pneumatic power unit (52) builds against the driving assembly (12); and
(d) overcoming with the predetermined amount of pressure an opposing force created by the driving assembly (12);
wherein at least one path (50) provides fluid communication of pressure from the power unit (52) to the locking means (18) wherein the locking means (18) is configured to release the driving means (12) when the power unit (52) builds a predeterminded amount of hydraulic or pneumatic pressure in the at least one path.

25. The method of claim 28 or claim 29, further comprising releasing the driving assembly (12) from the lock assembly (18) once the predetermined amount of pressure has been built against the driving assembly.

26. The method of any one of claims 24 to 25, further comprising relocking the driving assembly (12) into the lock assembly (18).

27. The method of claim 26, wherein relocking the driving assembly (12) into the lock assembly (18) comprises:
(a) supplying the pressure to the driving assembly;
(b) overcoming the opposing force created by the driving assembly; and
(c) realigning the driving assembly into the lock assembly.

28. The method of claim 26 or claim 27, wherein relocking the driving assembly (12) into the lock assembly (12) further comprises the step of drawing the substance (14) into the injector assembly (10).

29. The method of any one of claims 24 to 27, further comprising drawing a substance (14) into the injector assembly (10).

30. The method of any one of claims 24 to 29, wherein increasing pressure against the driving assembly (12) comprises supplying a compressed gas to a cavity (A) near the driving assembly.

31. The method of claim 30, wherein supplying a compressed gas to the cavity (A) near the driving assembly (12) comprises triggering a release of the compressed gas from a pneumatic power unit (52), the pneumatic power unit communicating with the cavity near the driving assembly.

32. The method of any one of claims 25 to 31, further comprising changing the dosage amount of the substance in the injector assembly (10).

33. The method of any of claims 30 to 32, wherein the predetermined amount of pressure comprises at least approximately 70% of the operation pressure of a compressed gas.

## Patentansprüche

1. Jetinjektoranordnung (10), umfassend:
(a) Antriebsmittel (12) zum Injizieren einer Substanz (14) in ein Subjekt (16);
(b) Verriegelungsmittel (18) zum Zurückhalten der Antriebsmittel (12); und
(c) Mittel zum Freigeben (20) der zurückgehaltenen Antriebsmittel (12) von den Verriegelungsmitteln (18);
wobei die Mittel zum Freigeben (20) der zurückgehaltenen Antriebsmittel (12) von den Verriegelungsmitteln eine Powereinheit (52) umfassen, die (a) wenigstens eine hydraulische oder pneumatische Druckeinheit umfasst, (b) einen ersten Pfad (50) umfasst, der die fluide Kommunikation von hydraulischem oder pneumatischem Druck von der Powereinheit (52) zu den Verriegelungsmitteln (18) bereitstellt, wobei die Verriegelungsmittel (18) ausgestaltet sind, die Antriebsmittel (12) freizugeben, wenn die Powereinheit (52) einen hydraulischen oder pneumatischen Druck einer vorbestimmten Größe in dem ersten Pfad (50) aufbaut, **gekennzeichnet durch** (c) einen zweiten Pfad (51), der eine fluide Kommunikation von hydraulischem oder pneumatischem Druck von der Powereinheit (52) zu den Antriebsmitteln (12) bereitstellt, wobei die Antriebsmittel (12) in eine verriegelte Position zurückgeführt werden, um freigegeben zu werden.

2. Injektoranordnung nach Anspruch 1, wobei die Antriebsmittel (12) einen Kolben (28) umfassen.

3. Injektoranordnung nach Anspruch 1 oder 2, wobei die Antriebsmittel eine Feder (32) umfassen.

4. Injektoranordnung nach einem der vorhergehenden Ansprüche, wobei die Antriebsmittel einen Kolben (28) und eine Feder (32) umfassen.

5. Injektoranordnung nach einem der vorhergehenden Ansprüche, wobei die Verriegelungsmittel (18) wenigstens eine Walze (38) umfassen.

6. Injektoranordnung nach einem der vorhergehenden Ansprüche, wobei die Verriegelungsmittel (18) wenigstens eine Walzenarretierung (46) umfassen.

7. Injektoranordnung nach einem der vorhergehenden Ansprüche, wobei die Verriegelungsmittel (18) eine ringförmige Nut (42) umfassen.

8. Injektoranordnung nach einem der vorhergehenden Ansprüche, wobei die Verriegelungsmittel (18) wenigstens eine Walze (38), wenigstens eine Walzenarretierung (46) und eine ringförmige Nut (42) umfassen.

9. Injektoranordnung nach einem der vorhergehenden Ansprüche, wobei ferner eine erste Komponente (22) umfasst wird.

10. Injektoranordnung nach Anspruch 9, wobei die Antriebsmittel (12) und die Verriegelungsmittel (18) in der ersten Komponente (22) angeordnet sind.

11. Injektoranordnung nach einem der vorhergehenden Ansprüche, wobei die Verriegelungsmittel (18) ferner ausgestaltet sind, die Antriebsmittel (12) in einer verriegelten Position und/oder einer nicht verriegelten Position in Eingriff zu nehmen.

12. Injektoranordnung nach einem der Ansprüche 8 bis 11, wobei die wenigstens eine Walzenarretierung (46) ausgestaltet ist, die wenigstens eine Walze (38) in der ringförmigen Nut (42) in einer verriegelten Position zu halten.

13. Injektoranordnung nach einem der vorhergehenden Ansprüche, wobei die Powereinheit (52) ausgestaltet ist, einen Druck auf die Antriebsmittel (12) auszuüben.

14. Injektoranordnung nach Anspruch 9 oder 10, wobei die erste Komponente (22) umfasst:
(i) eine Kolbenanordnung, die einen Kolben (28) und die Feder (32) umfasst;
(ii) eine Verriegelungsanordnung (18), die wenigstens eine Walze (38), wenigstens eine Walzenarretierung (46) und eine ringförmige Nut (42) umfasst, wobei die Verriegelungsanordnung ausgestaltet ist, die Kolbenanordnung in Eingriff zu nehmen; und
(iii) eine Injektionskammer (11),
wobei die Kolbenanordnung ausgestaltet ist, eine Substanz aus der Injektionskammer (11) herauszubefördern.

15. Injektoranordnung nach Anspruch 14, wobei eine pneumatische Druckeinheit (52) abnehmbar über wenigstens einen Pfad (50) an die erste Komponente angebracht ist.

16. Injektoranordnung nach einem der Ansprüche 7 bis 15, wobei der Kolben (28) ferner die ringförmige Nut (42) umfasst.

17. Injektoranordnung nach einem der Ansprüche 7 bis 16, wobei die wenigstens eine Walzenarretierung (46) ausgestaltet ist, die wenigstens eine Walze (38) in der ringförmigen Nut (42) zu halten.

18. Injektoranordnung nach einem der Ansprüche 15 bis 17, wobei die pneumatische Druckeinheit (52) ausgestaltet ist, einen Druck auf die Kolbenanordnung auszuüben.

19. Injektoranordnung nach einem der Ansprüche 15 bis 18, wobei die pneumatische Druckeinheit (52) eine Auslassanordnung (71) umfasst, die ausgestaltet ist, komprimierte Luft aus der pneumatischen Druckeinheit zu entlassen.

20. Injektoranordnung nach Anspruch 19, wobei die Auslassanordnung (71) einen Kanal (70), wenigstens einen Dichtungsring (74) und wenigstens eine Öffnungsplatte (72) umfasst.

21. Injektoranordnung nach einem der Ansprüche 14 bis 20, wobei die erste Komponente (22) ferner eine Schutzkappe (98) umfasst.

22. Injektoranordnung nach einem der Ansprüche 14 bis 21, wobei die erste Komponente (22) ferner eine Dosierungseinheit (82) umfasst, die ausgestaltet ist, das Zuführen einer vorbestimmten Menge einer Substanz anzupassen.

23. Injektoranordnung nach einem der Ansprüche 13 bis 22, wobei die vorbestimmte Größe des Druckes wenigstens ungefähr 70% des Betriebsdruckes eines komprimierten Gases entspricht.

24. Verfahren zum Verhindern einer vorzeitigen Injektion einer Substanz von einer Injektoranordnung, wobei es sich bei der Injektoranordnung um die Injektoranordnung nach Anspruch 1 handelt, umfassend:
(a) Verriegeln einer Antriebseinheit (12) in einer Verriegelungsanordnung (18);
(b) Steigern des Druckes von einer pneumatischen Powereinheit (52) auf die Antriebsanordnung (12);
(c) Zurückhalten der Antriebsanordnung (12) in der Verriegelungsanordnung (18) bis ein Druck einer vorbestimmten Größe von der pneumatischen Powereinheit (52) auf die Antriebsanordnung (12) ausgebildet worden ist; und
(d) Überwinden einer entgegen gerichteten Kraft, die durch die Antriebsanordnung (12) erzeugt wird, mit dem Druck einer vorherbestimmten Größe;
wobei wenigstens ein Pfad (50) eine fluide Kommunikation von Druck von der Powereinheit (52) zu den Verriegelungsmitteln (18) bereitstellt, wobei die Verriegelungsmittel (18) ausgestaltet sind, die Antriebsmittel (12) freizugeben, wenn die Powereinheit (52) in dem wenigstens einen Pfad einen hydraulischen oder pneumatischen Druck einer vorbestimmten Größe aufbaut.

25. Verfahren nach Anspruch 28 oder 29, wobei das Verfahren ferner das Freigeben der Antriebsanordnung (12) von der Verriegelungsanordnung (18) umfasst, sobald der Druck einer vorbestimmten Größe auf die Antriebsanordnung aufgebaut worden ist.

26. Verfahren nach einem der Ansprüche 24 oder 25, wobei das Verfahren ferner das erneute Verriegeln der Antriebsanordnung (12) in der Verriegelungsanordnung (18) umfasst.

27. Verfahren nach Anspruch 26, wobei das erneute Verriegeln der Antriebsanordnung (12) in der Verriegelungsanordnung (18) umfasst:
(a) Zuführen des Druckes zu der Antriebsanordnung;
(b) Überwinden der entgegen gerichteten Kraft, die durch die Antriebsanordnung erzeugt wird; und
(c) erneutes Ausrichten der Antriebsanordnung in der Verriegelungsanordnung.

28. Verfahren nach Anspruch 26 oder 27, wobei das erneute Verriegeln der Antriebsanordnung (12) in der Verriegelungsanordnung (12) ferner den Schritt umfasst, die Substanz (14) in die Injektoranordnung (10) einzuziehen.

29. Verfahren nach einem der Ansprüche 24 bis 27, wobei das Verfahren ferner das Einziehen einer Substanz (14) in die Injektoranordnung (10) umfasst.

30. Verfahren nach einem der Ansprüche 24 bis 29, wobei das Steigern des Druckes auf die Antriebsanordnung (12) das Zuführen eines komprimierten Gases zu einer Kavität (A) in der Nähe der Antriebsanordnung umfasst.

31. Verfahren nach Anspruch 30, wobei das Zuführen eines komprimierten Gases zu der Kavität (A) in der Nähe der Antriebsanordnung (12) das Triggern eine Freigabe des komprimierten Gases von einer pneumatischen Powereinheit (52) umfasst, wobei die pneumatische Powereinheit mit der Kavität in der Nähe der Antriebsanordnung kommuniziert.

32. Verfahren nach einem der Ansprüche 25 bis 31, wobei das Verfahren ferner das Verändern der Dosierungsmenge der Substanz in der Injektoranordnung (10) umfasst.

33. Verfahren nach einem der Ansprüche 30 bis 32, wobei die vorherbestimmte Größe des Druckes wenigstens ungefähr 70% des Betriebsdruckes eines komprimierten Gases beträgt.

## Revendications

1. Ensemble d'injecteur sans aiguille (10) comprenant :
(a) des moyens d'entraînement (12) pour injecter une substance (14) dans un sujet (16) ;
(b) des moyens de verrouillage (18) pour retenir les moyens d'entraînement (12) ; et
(c) des moyens pour libérer (20) les moyens d'entraînement (12) retenus, des moyens de verrouillage (18) ;
les moyens pour libérer (20) les moyens d'entraînement (12) retenus, des moyens de verrouillage comprennent une unité de puissance (52) qui comprend (a) au moins une unité de pression hydraulique ou pneumatique, (b) un premier passage (50) qui assure la communication de fluide de pression hydraulique ou pneumatique de l'unité de puissance (52) aux moyens de verrouillage (18), les moyens de verrouillage (18) étant configurés pour libérer les moyens d'entraînement (12) lorsque l'unité de puissance (52) accumule une quantité prédéterminée de pression hydraulique ou pneumatique dans le premier passage (50), **caractérisé par** (c) un second passage (51) qui assure la communication de fluide de pression hydraulique ou pneumatique de l'unité de puissance (52) aux moyens d'entraînement (12), les moyens d'entraînement (12) revenant dans une position verrouillée pour être libérés.

2. Ensemble d'injecteur selon la revendication 1, dans lequel les moyens d'entraînement (12) comprennent un piston (28).

3. Ensemble d'injecteur selon la revendication 1 ou la revendication 2, dans lequel les moyens d'entraînement comprennent un ressort (32).

4. Ensemble d'injecteur selon l'une quelconque des revendications précédentes, dans lequel les moyens d'entraînement comprennent un piston (28) et un ressort (32).

5. Ensemble d'injecteur selon l'une quelconque des revendications précédentes, dans lequel les moyens de verrouillage (18) comprennent au moins un rouleau (38).

6. Ensemble d'injecteur selon l'une quelconque des revendications précédentes, dans lequel les moyens de verrouillage (18) comprennent au moins un dispositif de retenue (46) de rouleau.

7. Ensemble d'injecteur selon l'une quelconque des revendications précédentes, dans lequel les moyens de verrouillage (18) comprennent une gorge annulaire (42).

8. Ensemble d'injecteur selon l'une quelconque des revendications précédentes, dans lequel les moyens de verrouillage (18) comprennent au moins un rouleau (38), au moins un dispositif de retenue (46) de rouleau et une gorge annulaire (42).

9. Ensemble d'injecteur selon l'une quelconque des revendications précédentes, comprenant en outre un premier composant (22).

10. Ensemble d'injecteur selon la revendication 9, dans lequel les moyens d'entraînement (12) et les moyens de verrouillage (18) sont situés dans le premier composant (22).

11. Ensemble d'injecteur selon l'une quelconque des revendications précédentes, dans lequel les moyens de verrouillage (18) sont en outre configurés pour venir en prise des moyens d'entraînement (12) dans au moins l'une parmi une position verrouillée et une position déverrouillée.

12. Ensemble d'injecteur selon l'une quelconque des revendications 8 à 11, dans lequel le au moins un dispositif de retenue (46) de rouleau est adapté pour maintenir au moins un rouleau (38) dans la gorge annulaire (42) dans une position verrouillée.

13. Ensemble d'injecteur selon l'une quelconque des revendications précédentes, dans lequel l'unité de puissance (52) est adaptée pour appliquer une pression sur les moyens d'entraînement (12).

14. Ensemble d'injecteur selon la revendication 9 ou la revendication 10, dans lequel le premier composant (22) comprend :
(i) un ensemble de piston comprenant le piston (28) et le ressort (32) ;
(ii) un ensemble de verrouillage (18) comprenant au moins un rouleau (38), au moins un dispositif de retenue (46) de rouleau, et une gorge annulaire (42), l'ensemble de verrouillage étant configuré pour venir en prise avec l'ensemble de piston ; et
(iii) une chambre d'injection (11),
l'ensemble de piston étant configuré pour entraîner une substance hors de la chambre d'injection (11).

15. Ensemble d'injecteur selon la revendication 14, dans lequel une unité de pression pneumatique (52) est fixée de manière détachable au premier composant par au moins un passage (50).

16. Ensemble d'injecteur selon l'une quelconque des revendications 7 à 15, dans lequel le piston (28) comprend en outre la gorge annulaire (42).

17. Ensemble d'injecteur selon l'une quelconque des revendications 7 à 16, dans lequel le au moins un dispositif de retenue (46) de rouleau est adapté pour maintenir le au moins un rouleau (38) dans la gorge annulaire (42).

18. Ensemble d'injecteur selon l'une quelconque des revendications 15 à 17, dans lequel l'unité de pression pneumatique (52) est adaptée pour appliquer une pression sur l'ensemble de piston.

19. Ensemble d'injecteur selon l'une quelconque des revendications 15 à 18, dans lequel l'unité de pression pneumatique (52) comprend un ensemble d'échappement (71) adapté pour libérer l'air comprimé de l'unité de pression pneumatique.

20. Ensemble d'injecteur selon la revendication 19, dans lequel l'ensemble d'échappement (71) comprend un canal (70), au moins un joint (74) et au moins une plaque à orifice (72).

21. Ensemble d'injecteur selon l'une quelconque des revendications 14 à 20, dans lequel le premier composant (22) comprend en outre un capuchon de protection (98).

22. Ensemble d'injecteur selon l'une quelconque des revendications 14 à 21, dans lequel le premier composant (22) comprend une unité de dosage (82) configurée pour ajuster l'administration d'une quantité prédéterminée d'une substance.

23. Ensemble d'injecteur selon l'une quelconque des revendications 13 à 22, dans lequel la quantité prédéterminée de pression représente au moins approximativement 70% de la pression de fonctionnement d'un gaz comprimé.

24. Procédé permettant d'empêcher l'injection prématurée d'une substance depuis un ensemble d'injecteur, dans lequel l'ensemble d'injecteur est l'ensemble d'injecteur de la revendication 1, comprenant les étapes consistant à :
(a) verrouiller un ensemble d'entraînement (12) dans un ensemble de verrouillage (18) ;
(b) augmenter la pression provenant d'une unité de puissance pneumatique (52) contre l'ensemble d'entraînement (12) ;
(c) retenir l'ensemble d'entraînement (12) dans l'ensemble de verrouillage (18) jusqu'à ce qu'une quantité prédéterminée de pression provenant de l'unité de puissance pneumatique (52) s'accumule contre l'ensemble d'entraînement (12) ; et
(d) surmonter, avec la quantité prédéterminée de pression, une force d'opposition créée par l'ensemble d'entraînement (12) ;
au moins un passage (50) assurant la communication de fluide de l'unité de puissance (52) aux moyens de verrouillage (18), les moyens de verrouillage (18) étant configurés pour déverrouiller les moyens d'entraînement (12) lorsque l'unité de puissance (52) accumule une quantité prédéterminée de pression hydraulique ou pneumatique dans le au moins un passage.

25. Procédé selon la revendication 28 ou la revendication 29, comprenant en outre l'étape consistant à déverrouiller l'ensemble d'entraînement (12) de l'ensemble de verrouillage (18) une fois que la quantité prédéterminée de pression s'est accumulée contre l'ensemble d'entraînement.

26. Procédé selon l'une quelconque des revendications 24 à 25, comprenant en outre l'étape consistant à verrouiller à nouveau l'ensemble d'entraînement (12) dans l'ensemble de verrouillage (18).

27. Procédé selon la revendication 26, dans lequel l'étape consistant à verrouiller à nouveau l'ensemble d'entraînement (12) dans l'ensemble de verrouillage (18) comprend les étapes consistant à :
(a) alimenter en pression l'ensemble d'entraînement ;
(b) surmonter la force d'opposition créée par l'ensemble d'entraînement ; et
(c) aligner à nouveau l'ensemble d'entraînement dans l'ensemble de verrouillage.

28. Procédé selon la revendication 26 ou la revendication 27, dans lequel l'étape consistant à verrouiller à nouveau l'ensemble d'entraînement (12) dans l'ensemble de verrouillage (12) comprend en outre l'étape consistant à aspirer la substance (14) dans l'ensemble d'injecteur (10).

29. Procédé selon l'une quelconque des revendications 24 à 27, comprenant en outre l'étape consistant à aspirer une substance (14) dans l'ensemble d'injecteur (10).

30. Procédé selon l'une quelconque des revendications 24 à 29, dans lequel l'étape consistant à augmenter la pression contre l'ensemble d'entraînement (12) comprend l'étape consistant à alimenter en gaz comprimé une cavité (A) située à proximité de l'ensemble d'entraînement.

31. Procédé selon la revendication 30, dans lequel l'étape consistant à alimenter en gaz comprimé la cavité (A) à proximité de l'ensemble d'entraînement (12) comprend l'étape consistant à déclencher une libération du gaz comprimé provenant d'une unité de puissance pneumatique (52), l'unité de puissance pneumatique communiquant avec la cavité située à proximité de l'ensemble d'entraînement.

32. Procédé selon l'une quelconque des revendications 25 à 31, comprenant en outre l'étape consistant à changer la quantité de dosage de la substance dans l'ensemble d'injecteur (10).

33. Procédé selon l'une quelconque des revendications 30 à 32, dans lequel la quantité prédéterminée de pression comprend au moins approximativement 70% de la pression de fonctionnement d'un gaz comprimé.
